# EUROPEAN PATENT SPECIFICATION

(11) **EP 1 075 517 B1**
(45) Date of publication and mention of the grant of the patent: **26.07.2006**
(21) Application number: 99919447.5
(22) Date of filing: 06.05.1999
(51) Int. Cl.: C12N 15/13, C12N 15/62, C12N 5/10, C07K 14/705, C07K 14/725, C07K 14/73, C07K 16/28

(54) **CHIMERIC RECEPTORS**
CHIMÄRE REZEPTOREN
RECEPTEURS CHIMERES

(30) Priority: 06.05.1998 GB 9809658
(43) Date of publication of application: 14.02.2001
(73) Proprietor: CELLTECH THERAPEUTICS LIMITED, Slough, Berkshire SL1 3WE (GB)
(72) Inventor: LAWSON, Alastair, David, Griffiths, Alresford Hampshire SO2 0NX (GB); FINNEY, Helene, Margaret, Maidenhead Berkshire SL6 4DQ (GB)
(74) Representative: Mercer, Christopher Paul
(86) International application number: PCT/GB1999/001417
(87) International publication number: WO 1999/057268

(56) References cited:
- WO-A-92/10591
- WO-A-92/15322
- WO-A-93/19163
- WO-A-95/02686
- WO-A-96/23814
- WO-A-96/24671
- WO-A-97/23613
- WO-A-99/00494
- GROSS G ET AL: "EXPRESSION OF IMMUNOGLOBULIN-T-CELL RECEPTOR CHIMERIC MOLECULES AS FUNCTIONAL RECEPTORS WITH ANTIBODY-TYPE SPECIFICITY" PROCEEDINGS OF THE NATIONAL ACADEMY OF SCIENCES OF USA, vol. 86, 1 December 1989 (1989-12-01), pages 10024-10028, XP002054291 ISSN: 0027-8424 cited in the application

## Description

This invention relates to chimeric receptors, to DNA coding therefor and to the use of the receptors in medicine.

Chimeric receptors have been designed to target cells such as T-cells to other cells expressing antigen on their cell surface. Binding of antigen to the receptor in the correct context triggers a series of intracellular events leading to activation of the receptor bearing cell. Activation may lead to an increase in proliferation; expression of cytokines, with for example pro or anti-inflammatory responses; stimulation of cytolytic activity, differentiation or other effector functions; antibody secretion; phagocytosis; tumour infiltration and/or increased adhesion. Clearly such activation may have therapeutic benefits and chimeric receptors which can facilitate this are of use in the treatment of a number of diseases or disorders.

Previously described chimeric receptors provide antigen recognition either in a single chain, for example as in a single chain, Fv or CD4, linked to an intracellular signalling region [Eshhar, Z *et al,* (1993) Proc. Natl. Acad. Sci. USA 90, 720; Stancovski, I *et al* (1993) J. Immunol. 151. 6577; Hwu, P *et al*, (1993) J. Exp. Med. 178. 361; Brocker, T *et al,* (1993) Eur. J. Immunol. 23, 1435; Moritz, D. *et al* (1994) Proc. Natl. Acad. Sci. USA, 91, 4318; Roberts, M. *et al*, (1994) Blood 84, 2878; Hwu, P *et al*, (1995) Cancer Res. 55 3369; Tran, A-C *et al* (1995) J. Immunol. 155, 1000; Hekele, A *et al* (1996) Int. J. Cancer 68, 232; Altenschmidt, U *et al* (1996) Clin. Cancer Res. 2, 1001; Brocker, T *et al* (1996) Eur. J. Immunol 26, 1770; Weitjens, M *et al* (1996) J. Immunol. 157, 836; Alvarez-Vallina, L and Hawkins, R E (1996) Eur. J. Immunol. 26, 2304], or in two chains as in V_{L-}TCRα with V_{H}-TCRβ or V_{H}-TCRα with V_{L}-TCRβ but with no intracellular signalling sequences attached [Kuwana, Y *et al*, (1987) Biochem. Biophys. Res. Commun. 149, 960; Gross, G *et al* (1989) Proct. Natl. Acad. Sci. USA 86, 10024].

The mechanisms by which such receptors convert the extracellular binding event into intracellular signalling are largely unclear, and are likely to involve clustering and association with endogenous cellular effector molecules. One disadvantage in their design is that there is no inherent mechanism to prevent constitutive activation in the absence of antigenic stimulation. This is undesirable since it can lead to inappropriate activation of a cell. Another problem with previously described chimeric receptors is that they are susceptible to signalling on binding soluble antigen. This limits their usefulness in the treatment of some disorders, for example in tumour therapy where many cell associated tumour antigens are also shed into the vascular system and can therefore induce inappropriate signalling by the chimeric receptor away from the tumour site.

The present invention provides an improved chimeric receptor which minimises constitutive activation in the absence of antigen and is less readily triggered by soluble antigen than previously described designs. In one particularly advantageous form the receptor according to the invention includes a mechanism whereby multiple signalling components can be localised on binding of antigen to act cooperatively to efficiently generate an intracellular signal.

The improved chimeric receptors according to the invention generally feature two or more polypeptide chains each of which contains an extracellular ligand association domain attached to a signalling domain through a transmembrane and optionally one or more spacer domains. The ligand association domains are capable of acting cooperatively with each other in the presence of ligand to form a ligand binding site. Each chain may be expressed so that it locates in a cell membrane with an orientation in which the association domain is extracellular and the signalling domain is intracellular. By careful selection of the ligand association domains and non-associating spacer and/or transmembrane domains each polypeptide chain can be expressed independently and will remain largely unassociated with the other(s) in the absence of ligand. The presence of ligand, especially cell surface expressed ligand induces a stable interaction between the ligand association domains, specifically stabilising a close spatial proximity of the polypeptide chains, and facilitating interaction between the intracellular signalling domains. The signalling domains can be selected such that one forms a substrate for the other, thus increasing the efficiency of the signalling event.

The chimeric receptor according to the invention can be expressed in a host cell transformed with DNA coding for each polypeptide chain. Thus according to one aspect of the invention we provide DNA coding for a chimeric receptor containing two or more independent polypeptide chains each of said chains comprising in a N- to C-terminus sequence:
(1) an extracellular ligand association domain;
(2) a transmembrane domain; and
(3) one or more intracellular domains;
provided that at least two of said domains in one chain are not naturally fused to each other.

For the avoidance of doubt, the term "not naturally fused" as used herein is intended to mean that two or more domains are not linked in a way which generates a polypeptide found in nature. Clearly, in this way, naturally occurring receptors are intended to be excluded from the invention. Providing that at least two domains are not naturally fused in this way other domains, where desired, may be linked in a naturally occurring arrangement.

As used herein the term extracellular ligand association domain is intended to mean any oligo- or polypeptide which is capable of interacting with cell surface molecules expressed on a target or host cell.

Thus the domain may be chosen to recognise a surface marker expressed on target cells associated with a disease state such as for example those associated with virally infected cells; bacterially infected cells; cancer cells, such as the bombesin receptor expressed on lung tumour cells, carcinoembryonic antigen, polymorphic epithelial mucin and CD33; cell surface adhesion molecules; inflammatory cells present in autoimmune disease; or a T-cell receptor or antigen giving rise to autoimmunity.

Alternatively, the association ligand domain may be chosen such that it interacts with one or more of the other ligand association domains of the chimeric receptor expressed by the host cell to achieve multiply-associated domains capable of recognising a surface marker expressed on a target cell as just described.

Particularly useful ligand association domains include parts of receptors associated with binding to cell surface associated molecules and especially include an antibody variable region (V_{H} or V_{L}) domain, a T-cell receptor variable region domain (TCRα, TCRβ, TCRγ, TCRδ) or a chain selected from CD8α, CD8β, CD11a, CD11b, CD11c, CD18, CD29, CD49a, CD49b, CD49c, CD49d, CD49e, CD49f, CD61, CD41 or CD51. Fragments of these domains and chains may be used where appropriate.

Each association domain in the chimeric receptor may be the same, although desirably the association domains are structurally different. In one preferred arrangement, the domains are able to cooperatively with each other to form a ligand binding site. Particular examples include a V_{H} domain paired with a V_{L} domain, two or more TCRα, TCRβ, TCFγ, and/or TCRδ domains, a CD8α or β homo- or heterodimer, CD18 paired with one or more of CD11a, b, or c, CD29 paired with one or more of CD49a, b, c, d, e, or f, and CD61 paired with CD41c and/or CD51.

In binding to the ligand each association domain moves to form a ligand binding site and in so doing establishes a close spatial proximity of the intracellular domains which form the C-terminal regions of the polypeptide chains which constitute the chimeric receptor. Particularly useful ligand association domains include antibody V_{H} and V_{L} domains and fragments thereof, especially in a two chain receptor where one of the association domains is a V_{H} domain or a fragment thereof and the other is a V_{L} domain or a fragment thereof.

As used herein the term intracellular domain is intended to mean any oligo- or polypeptide which can participate in the transduction of a signal which results in direct or indirect activation of one or more intracellular messenger systems. Particular intracellular messenger systems include for example one or more kinase pathways such as those involving tyrosine kinase, protein kinase C or MAP kinase; G-protein or phospholipase mediated pathways; calcium mediated pathways; and pathways involving synthesis of a cytokine such as an interleukin e.g. IL-2, including NFAT, and cAMP mediated pathways.

Each intracellular domain may be derived from one or more naturally occurring polypeptide signalling sequences. Examples of suitable sequences include, for example sequences derived from the T-cell receptor such as all or part of the zeta, eta or epsilon chain; CD28; CD4; CD8; the γ chain of a Fc receptor; or signalling components from a cytokine receptor e.g. interleukin, TNF and interferon receptors, a colony stimulating factor receptor e.g. GMCSF, a tyrosine kinase e.g. ZAP-70, fyn, Ick, Itk and syk and binding domains thereof; an adhesion molecule e.g. LFA-1 and LFA-2, B29, MB-1, CD3 delta, CD3 gamma, CD5 or CD2.

At least one component in each intracellular domain will be capable of interacting cooperatively with one or more other components in other intracellular domains. Cooperative interaction includes for example association of two or more components to form a substrate capable of participating in one of the intracellular messenger systems described above. Preferably however cooperative interaction means one of the components acting as a substrate for one or more others such that the substrate initiates a signalling event. This can either lead to an activation or down-regulation of signalling cascade. A particular example of this type of cooperative interaction may be obtained when one of the components in an intracellular domain is derived from a CD4 intracellular chain containing the Ick binding domain and a component in the other intracellular domain is a zeta chain derived from the T-cell receptor. Binding of ligand to the chains of the chimeric receptor causes association of lck with zeta facilitating phosphorylation of the zeta ARAM tyrosine residues, an early event in signal generation.

The transmembrane domain in each polypeptide chain of the chimeric receptor generally serves to anchor each chain to the cell membrane of the host cell. Transmembrane domains may in general be any oligo- or polypeptide and may be derived from a wide variety of sources such as all or part of the alpha, beta or zeta chain of the T-cell receptor, CD28, CD8, CD4, CD3ε, CD45 and members of the tetraspan family e.g. CD9, CD37 a cytokine receptor, e.g. an interleukin receptor, TNF receptor, or interferon receptor, or a colony stimulating factor receptor, e.g. GMCSF.

Whatever the derivation of each transmembrane domain it will be desirably chosen or modified to minimise its constitutive association with any other domain in the chimeric receptor but to allow association of the receptor polypeptide chains when ligand is bound by one or more extracellular association domains. This reduces undesirable random signal generation by ensuring that the intracellular domains only interact when ligand is bound by the extracellular association domains. This forms an important aspect of the design of the receptors of the invention.

In addition to the selection of appropriate transmembrane domains, the ability of each receptor polypeptide chain to remain unassociated except in the presence of bound ligand may be enhanced by incorporating a spacer region between each extracellular association domain and transmembrane domain. Thus, according to a preferred aspect of the invention we provide DNA coding for a chimeric receptor containing two or more independent polypeptide chains, each of said chains comprising in N- to C-terminus sequence:
(1) an extracellular ligand association domain;
(2) a spacer domain;
(3) a transmembrane domain; and
(4) one or more intracellular domains; provided that at least two of said domains in one chain are not naturally fused to each other.

The term spacer domain as used herein generally means any oligo- or polypeptide serving to link the association and transmembrane domains in each chain. Spacer domains may for example comprise up to 300 amino acids, preferably 20 to 100 amino acids and most preferably 25 to 50 amino acids.

Spacers may be derived from all or part of naturally occurring molecules such as from all or part of the extracellular region of CD8, CD4 or CD28; or all or part of an antibody constant region, including the hinge region. All or part of natural spacing components between functional parts of intracellular signalling molecules, for example spacers between ITAMS (immunoreceptor tyrosine based activation motifs) may also be used. Alternatively the spacer may be a non-naturally occurring sequence.

In order to minimise the constitutive association of transmembrane and/or spacer domains, non-naturally associating domains may initially be selected and/or domains may be modified to reduce association. This may be achieved by deleting, changing or otherwise modifying amino acids of naturally occurring sequences in the transmembrane and/or spacer domains which have side chains capable of covalently or non-covalently interacting with the side chains of amino acids in the other chain. Particular examples of amino acids of these types include cysteine residues, charged amino acids or amino acids such as serine or threonine within potential glycosylation sites.

The DNA according to the invention will additionally contain coding sequences for a signal component for each of the chains of the chimeric receptor to enable each chain to be transported to the host cell membrane. Each signal will be attached to the N-terminus of the association domain of each chain. The signal component may be that naturally associated with the association domain or may be derived from other sources. Examples of secretion signals include immunoglobulin signal sequences.

The signal, association, spacer, transmembrane and intracellular domains of each chain in the chimeric receptor are preferably derived from or based on human sequences.

Particularly useful DNA according to the invention is that coding for a chimeric receptor containing two independent polypeptide chains as described herein. In receptors of this type, one of the chains preferably has a ligand association domain which is a V_{H} domain or a fragment thereof, and the other has a ligand association domain which is a V_{L} domain or a fragment thereof.

DNA coding sequences for use in the invention are widely available in the literature and from databases. The DNA may be obtained from readily available DNA sources for example commerically available cDNA or cDNA libraries using standard molecular biology and/or chemistry procedures, for example by use of the polymerase chain reaction (PCR), oligonucleotide directed mutagenesis or oligonucleotide directed synthesis techniques, enzymatic cleavage or enzymatic filling in of gapped oligonucleotides. Such techniques are described by Maniatis *et al* in Molecular Cloning, Cold Spring Harbor Laboratory, New York 1989, and in particular in the Examples hereinafter.

The DNA may be used in association with a carrier. The carrier may be a vector or other carrier suitable for introduction of the DNA *ex-vivo* or *in-vivo* into target cells and/or target host cells. Examples of suitable vectors include viral vectors such as retroviruses, adenoviruses, adenoassociated viruses, EBV, and HSV, and non-viral vectors, such as liposomal vectors and vectors based on DNA condensing agents for example cationic lipids such as those described in International Patent Specifications Nos. WO96/10038, WO97/18185, WO97/25329, WO97/30170 and WO97/31934. Where appropriate, the vector may additionally include promoter/regulatory sequences and/or replication functions from viruses such as retrovirus LTRs, AAV repeats, SV40 and hCMV promoters and/or enhancers, splicing and polyadenylation signals; EBV and BK virus replication functions. Tissue specific regulatory sequences such as the TCR-α promoter, E-selectin promoter and the CD2 promoter and locus control region may also be used. Alternatively the carrier may be an antibody.

Each DNA molecule coding for a polypeptide chain of the chimeric receptor may be incorporated into different carriers as described above. Preferably however the DNA is incorporated into the same carrier. For this the DNA may be located for example on separate plasmids or may be advantageously part of a single plasmid additionally containing one or more promoter and/or regulatory sequences and or replication functions as just described. Thus the invention extends to a plasmid comprising DNA coding for a chimeric receptor according to the invention. Particularly useful plasmids of this type include plasmid pHMF374 described in the Examples hereinafter and analogous plasmids containing other ligand association, spacer and/or transmembrane, and intracellular domains to those specified therein.

For *ex-vivo* use, the DNA of the invention may be introduced into effector cells removed from the target host using methods well known in the art e.g. transfection, transduction, biolistics, protoplast fusion, calcium phosphate precipitated DNA transformation, electroporation, cationic lipofection, or targeted liposomes. The effector cells are then reintroduced into the host using standard techniques.

A wide variety of target hosts may be employed according to the present invention such as, for example, mammals and, especially, humans.

Examples of suitable effector cells include cells associated with the immune system such as lymphocytes e.g. cytotoxic T-lymphocytes, tumour infiltrating lymphocytes, natural killer cells, neutrophils, basophils or T-helper cells; dendritic cells, B-cells, haemoatopaietic stem cells, macrophages, monocytes or NK cells. The use of cytotoxic T-lymphocytes is especially preferred.

The DNA according to the invention is particularly suitable for *in vivo* administration. It may be in one preferred example in the form of a targeted carrier system in which a carrier as described above is capable of directing the DNA to a desired effector cell. Particular examples of such targeted delivery systems include targeted-naked DNA, targeted liposomes encapsulating and/or complexed with the DNA, targeted retroviral systems and targeted condensed DNA such as protamine and polylysine condensed DNA.

Targeting systems are well known in the art and include using, for example, antibodies or fragments thereof against cell surface antigens expressed on target cells *in vivo* such as CD8; CD16; CD4; CD3; selectins e.g. E-selectin; CD5; CD7; CD34; activation antigens e.g. CD69 and IL-2R. Alternatively, other receptor - ligand interactions can be used for targeting e.g. CD4 to target HIV_{gp}160 - expressing target cells.

In general the use of antibody targeted DNA is preferred, particularly antibody targeted naked DNA, antibody targeted condensed DNA and especially antibody targeted liposomes. Particular types of liposomes which may be used include for example pH-sensitive liposomes where linkers cleaved at low pH may be used to link the antibody to the liposome. Cationic liposomes which fuse with the cell membrane and deliver the recombinant chimeric receptor DNA according to the invention directly into the cytoplasm may also be used. Liposomes for use in the invention may also have hydrophilic groups attached to their surface to increase their circulating half-life such as for example polyethylene glycol polymers. There are many examples in the art of suitable groups for attaching to liposomes or other carriers; see for example International Patent Specifications Nos. WO 88/04924, WO 90/09782, WO 91, 05545, WO 91/05546, WO 93/19738, WO 94/20073 and WO 94/22429. The antibody or other targeting molecule may be linked to the DNA, condensed DNA or liposome using conventional readily available linking groups and reactive functional groups in the antibody e.g. thiols, or amines and the like, and in the DNA or DNA containing materials.

Non-targeted carrier systems may also be used and in these targeted expression of the DNA is advantageous. Targeted expression of the DNA may be achieved for example by using T-cell specific promoter systems such as the zeta promoter and CD2 promoter and locus control region, CD4, CD8, TCRα and TCRβ promoters, cytokine promoters such as the IL2 promoter and the perforin promoter.

The DNA according to the invention may be used *ex vivo* and in a further aspect the invention provides effector cells transfected with DNA according to the invention. The effector cells may be any of those previously described above which are suitable for *ex vivo* use and are preferably T-cells most preferably cytotoxic T-cells.

The DNA according to the invention may take the form of a pharmaceutical composition. It may be a therapeutic or diagnostic composition and may take any suitable form suitable for administration. Preferably it will be in a form suitable for parenteral administration e.g. by injection or infusion, for example by bolus injection or continuous infusion or particle mediated injection. Where the composition is for injection or infusion, it may take the form of a suspension, solution or emulsion in an oily or aqueous vehicle and it may contain formulatory agents such as suspending, preservative, stabilising and/or dispersing agents. Alternatively, the composition may be in dry form, for reconstitution before use with an appropriate sterile liquid. For particle mediated administration the DNA may be coated on particles such as microscopic gold particles.

If the composition is suitable for oral administration the formulation may contain, in addition to the active ingredient, additives such as: starch - e.g. potato, maize or wheat starch or cellulose - or starch derivatives such as microcrystalline cellulose; silica; various sugars such as lactose; magnesium carbonate and/or calcium phosphate. It is desirable that, if the formulation is for oral administration it will be well tolerated by the patient's digestive system. To this end, it may be desirable to include in the formulation mucus formers and resins. It may also be desirable to improve tolerance by formulating the compositions in a capsule which is insoluble in the gastric juices. It may also be preferable to include the composition in a controlled release formulation.

The DNA according to the invention is of use in medicine and the invention extends to a method of treatment of a human or animal subject, the method comprising administering to the subject an effective amount of a DNA delivery system described above. The exact amount to be used will depend on the ages and condition of the patient, the nature of the disease or disorder and the route of administration, but may be determined using conventional means, for example by extrapolation of animal experiment derived data. In particular, for *ex vivo* use the number of transfected effector cells required may be established by *ex vivo* transfection and reintroduction into an animal model of a range of effector cell numbers.

Similarly the quantity of DNA required for *in vivo* use may be established in animals using a range of DNA concentrations.

The DNA according to the invention may be useful in the treatment of a number of diseases or disorders. Such diseases or disorders may include those described under the general headings of infectious diseases, e.g. HIV infection; inflammatory disease/autoimmunity e.g. rheumatoid arthritis, osteoarthritis, inflammatory bowel disease; cancer; allergic/atopic diseases e.g. asthma, eczema; congenital e.g. cystic fibrosis, sickle cell anaemia; dermatologic, e.g. psoriasis; neurologic, e.g. multiple sclerosis; transplants e.g. organ transplant rejection, graft-versus-host disease; metabolic/idiopathic disease e.g. diabetes.

The following Example illustrates the invention. In the Example the results show that the two chain chimeric receptor is not constitutively activated, in that no IL-2 was produced in the absence of target cells (HL60) or in the presence of cells not expressing specific antigen (NSO), and can be triggered to produce IL-2 only in the presence of cells expressing specific antigen (HL60 or NSO.CD33).

### EXAMPLE

### CONSTUCTION OF CHIMERIC RECEPTOR GENES

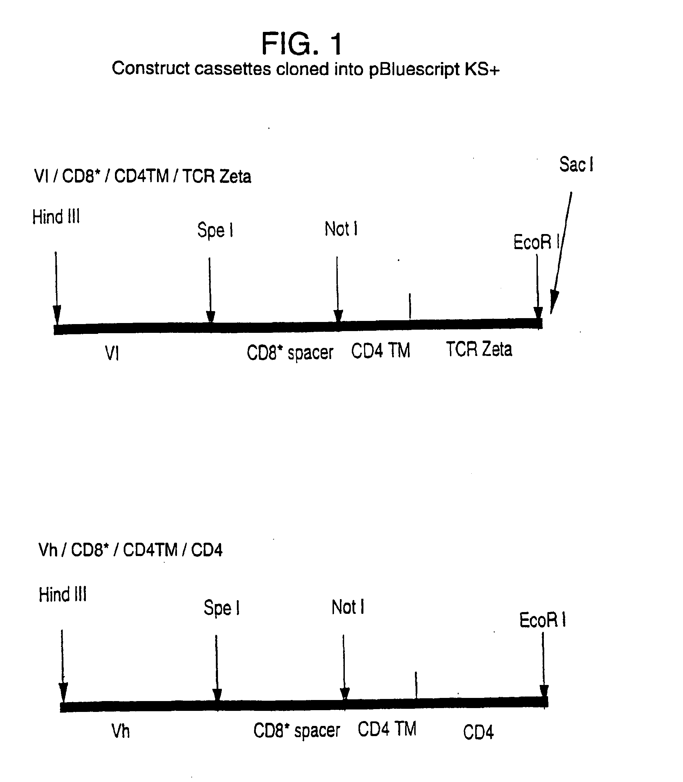

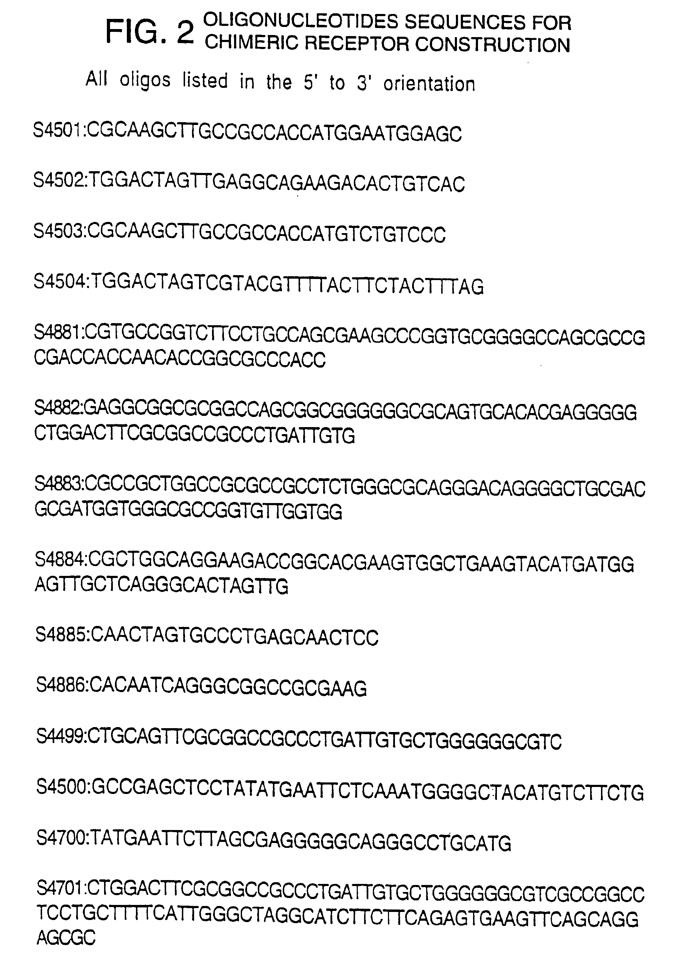

Each component of the chimeric receptor was either PCR cloned or PCR assembled by standard techniques (PCR Protocols, Innis *et al* (1990) Academic Press Inc.) and sub-cloned in a cassette format into pBluescript KS+ (Stratagene), see Figure 1. Oligonucleotides (oligos) are described in Figure 2.

### a) VI Cassette

The variable region of the light chain of the human engineered antibody, hP67 (engineerd according to International Patent Specification WO91/09967) was PCR cloned with oligos S4503 and S4504. S4503 introduces a 5' Hind III site and S4504 a 3' Spe I site. The PCR product was restricted with Hind III and Spe I and subcloned into pBluescript KS+.

### b) Vh Cassette

The variable region of the heavy chain of the human engineered antibody, hP67 (engineered according to International Patent Specification WO91/0997) was PCR cloned with oligos S4501 and S4502. S4501 introduces a 5' Hind III site and S4502 a 3' Spe I site. The PCR product was restricted with Hind III and Spe I and subcloned into pBluescript KS+.

### c) CD8* Spacer Cassette

The CD8* spacer cassete was PCR assembled using overlapping oligos: S4881, S4882, S4883, S4884, S4885 and S4886. The PCR product was restricted with Spe I and Not I and subcloned into pBluescript KS+.

### d) CD4 TM/CD4 Cassette

The CD4 transmembrane and intracellular components were PCR cloned from human Leukocyte cDNA (Clonetech) with oligos S4499 and S4500. S4499 introduces a 5' Not I site and S4500 introduces a 3'EcoR I and Sac I site. The PCR product was restricted with Not I and Sac I and subcloned into pBluescript KS+.

### e) CD4 TM / TCR Zeta Cassette

The intracellular component of TCR Zeta was PCR cloned from human Leukocyte cDNA (Clonetech) with oligos S4701 and S4700. S4701 is a long oligo which introduces both a 5' Not I site and the CD4 transmembrane component. S4700 introduces a 3' EcoR I site.

The PCR product was restricted with Not I and EcoR I and substituted for the CD4 TM / CD4 cassette in pBluescript KS+.

All of the above cassettes were sequenced (Applied Biosystems, Taq DyeDeoxy Terminator Cycle Sequencing, Part Number 901497) in pBluescript KS+ prior to cloning into expression vectors.

These cassettes were assembled using standard Molecular Biology techniques to construct the following Separate Chain chimeric receptors which when associated have the potential for human CD33 specificity.

### a) VH / CD8* / CD4 TM / CD4

The VH / CD8* / CD4 TM / CD4 chimeric receptor consists of the variable region of the heavy chain of the human engineered antibody P67 linked via and extracellular spacer based upon part of human CD8 hinge to the transmembrane and intracellular components of human CD4.

The extracellular spacer consists of residues 95 to 159 of human CD8 (with the following amino acid substitution:- Cys (143) changed to Ala to remove a potential disulphide bond and Thr (117, 118 and 119) changed to Gly, Ala, Gly respectively to reduce potential negative charge) followed by a Gly residue to introduce a restriction site [Sukhatme *et al,* (1985) Cell 40, 591-597). The CD4 transmembrane and intracellular component consists of residues 375 to 435 [Maddon *et al,* (1985) Cell, 42, 93-104].

### b) VI /CD8* / CD4 TM/TCR Zeta

The VI / CD8* / CD4 TM / TCR Zeta chimeric receptor consists of the variable region of the light chain of the human engineered antibody P67 linked via an extracellular spacer based upon part of human CD8 hinge to the transmembrane and intracellular components of human CD4.

The extracellular spacer consists of residues 95 to 159 of human CD8 (with the following amino acid substitution:- Cys (143) changed to Ala to remove a potential disulphide bond and Thr (117, 118 and 119) changed to Gly, Ala, Gly respectively to reduce potential negative charge) followed by a Gly residue to introduce a rest riction site [Sukhatme *et al* (1985) Cell, 40, 591-597]. The CD4 transmembrane component consists of residues 375 to 395 [Sukhatme *et al,* (1985), Cell, 40, 591-597]. The TCR Zeta intracellular component consist of residues 31 to 142 [Weissman *et al,* (1988) PNAS, 85, 9709-9713. Moingeon *et al* (1990) Eur. J. Immunol, 20, 1741-1745].

### ANALYSIS OF SEPARATE CHAIN CHIMERIC RECEPTORS EXPRESSED IN JURKAT CELLS

### a) Construction of expression plasmids

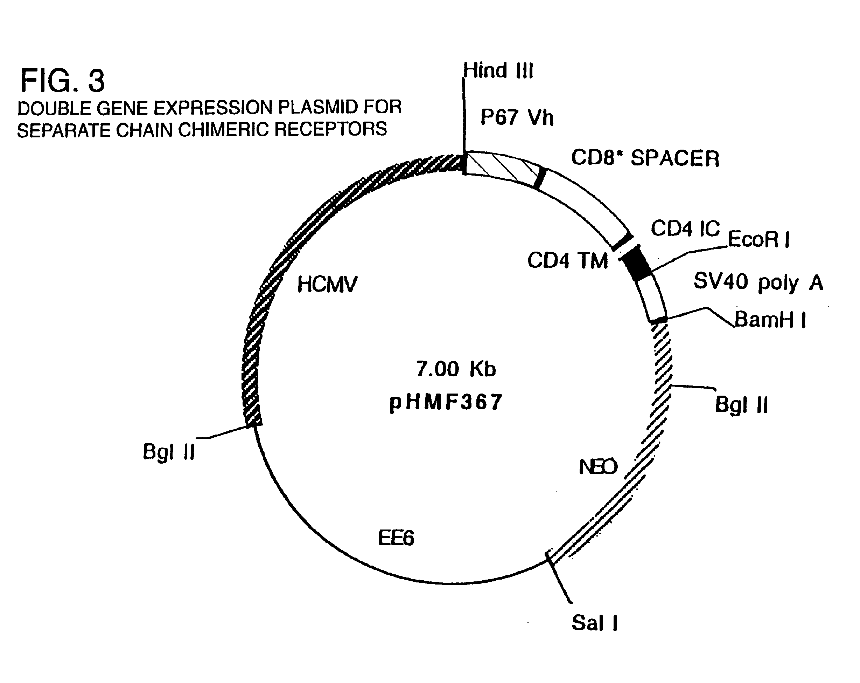

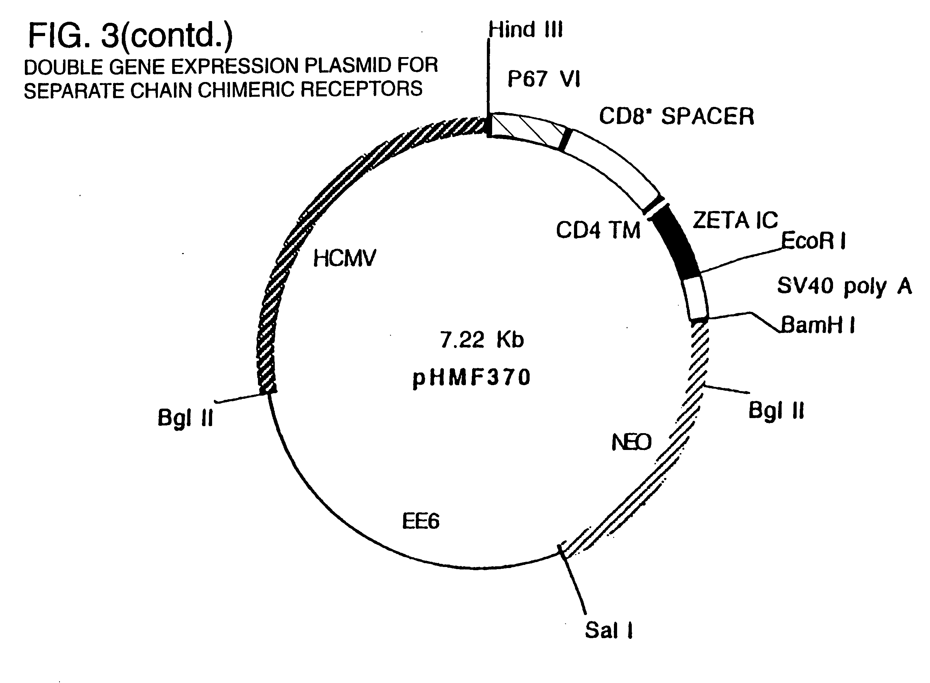

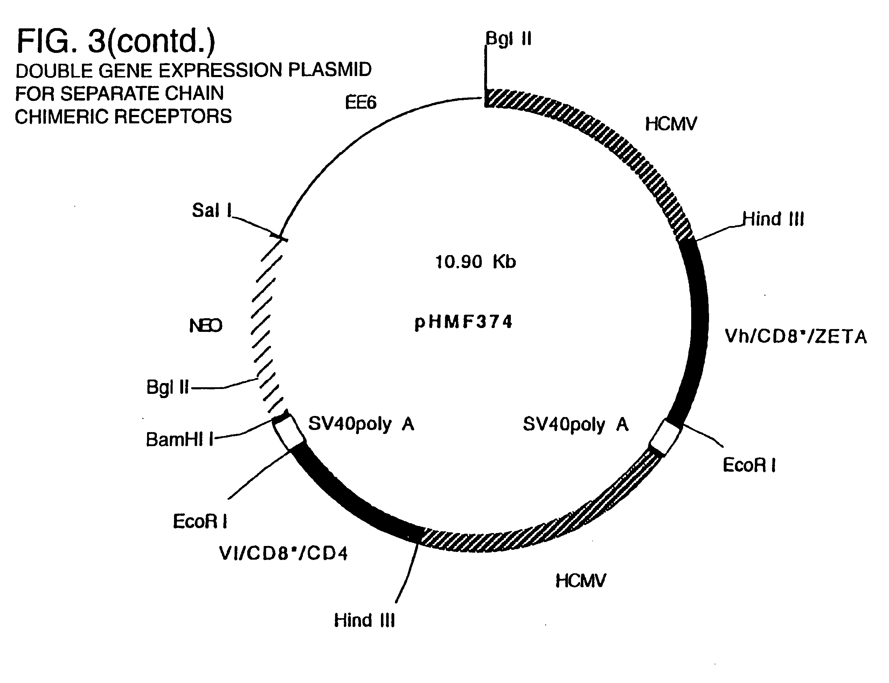

Chimeric receptor constructs were subcloned from pBluescript KS+ into the expression vector pEE6hCMV.ne [Bebbington (1991), Methods 2, 136-145] on a Hind III to EcoR I restriction fragment to generate plasmids pHMF367 and pHMF370 (see Figure 3). An expression vector was constructed expressing both separate chain chimeric receptor genes by subcloning a Bgl to BamH I fragment consisting of hCMV promoter, VI / CD8* / CD4 TM / TCR Zeta and SV40 poly A site into the BamH I site of pHMF367. This double receptor plasmid is pHMF374 (see Figure 3).

### b) Construction of Jurkat cell lines

Plasmids were linearised and transfected into Jurkat E6.1 cells (ECACC) by electroporation using a Bio-Rad Gene Pulser. 30µg of DNA per 1 x 10⁷ cells were given two pulses of 100v, 3µF in 1 ml PBS. Cells were left to recover overnight in non-selective media before being selected and cultured in media supplemented with the antibiotic G418 at 2mg/ml. After approximately four weeks cells were ready for IL-2 production analysis.

### c) Analysis of antigen-specific IL-2 production

1 x 10⁵ Jurkat cells expressing either control plasmid, pEE6hCMV.ne (J. control) or double receptor plasmid, pHMF374 (J.VL/VH) were incubated overnight with target cells at various effector (E): target cell (T) ratios in a 96 well plate (Falcon) at 37°C/8% CO₂.

Target cells used were : the human myelocytic cells line, HL60 which expresses CD33 or the mouse myeloma, NSO transfected with either a control plasmid or one expressing human CD33. After 20-24 hours cells were centrifuged and supernatant assayed for IL-2 (Quantikine kit, R & D Systems).

### RESULTS

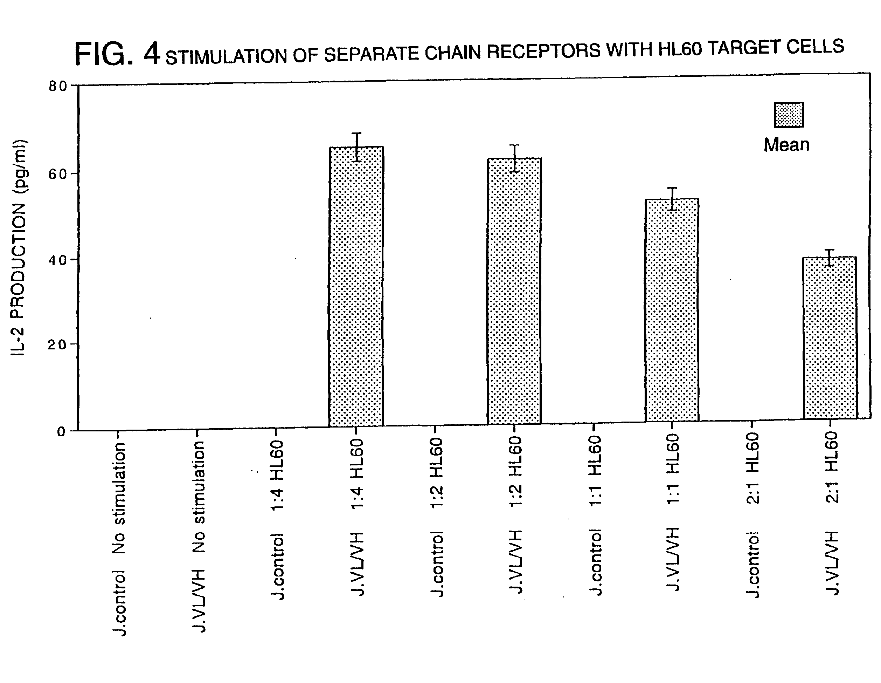

Figure 4 shows IL-2 production from Jurkat cells expressing VI / Vh separate chain chimeric receptors challenged with CD33 positive HL60 target cells. Jurkat cells expressing a control plasmid produced no IL-2 when incubated with HL60 cells, and Jurkat transfectants expressing the separate chain chimeric receptors did not constitutively produce IL-2 in the absence of target cells. IL-2 was specifically produced from transfectants on challenge with antigen bearing target cells, with the amount of IL-2 decreasing as the E : T ratio was increased.

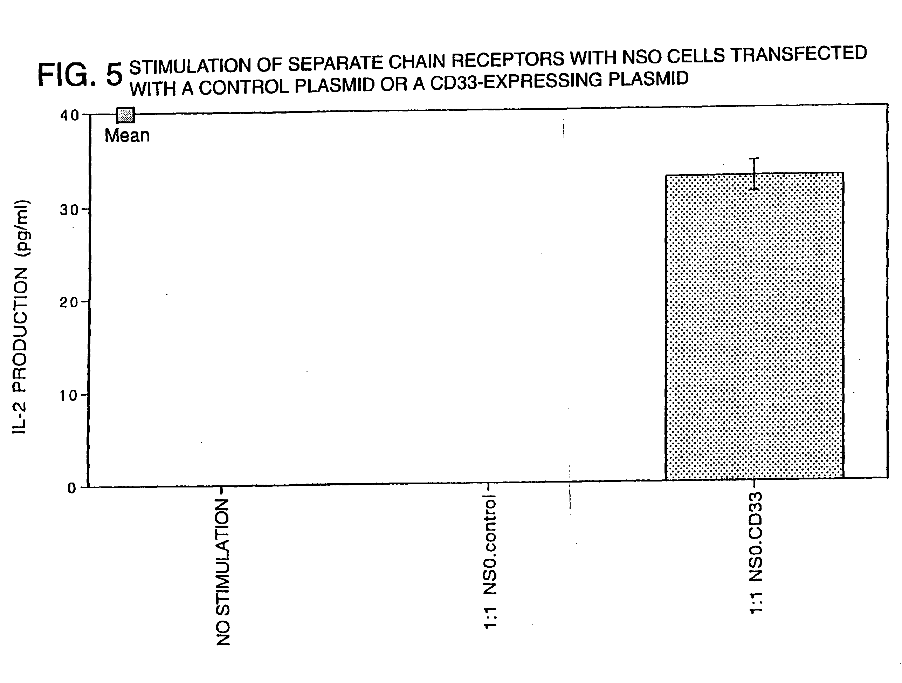

Figure 5 shows the antigen specificity of IL-2 production from Jurkat transfectants expressing separate chain chimeric receptors. NSO cells transfected with a control plasmid failed to elicit an IL-2 response from Jurkat cells expressing separate chain chimeric receptors, however on challenge with NSO cells that had been transfected with a CD33 plasmid and shown to express cell surface CD33, Jurkat transfectants expressing separate chain chimeric receptors produced IL-2.

## Claims

1. DNA coding for a chimeric receptor containing two independent polypeptide chains each of said chains comprising in a N- to C-terminus sequence:
(1) an extracellular ligand association domain;
(2) a spacer domain;
(3) a transmembrane domain; and
(4) one or more intracellular domains;
provided that:
the ligand association domain of one polypeptide chain of the chimeric receptor coded for is a V_{H} domain or a ligand binding fragment thereof and the ligand association domain of the other polypeptide chain of the chimeric receptor coded for is a V_{L} domain or a ligand binding fragment thereof;
at least two of said domains in one chain are not naturally fused to each other; and
the ligand association domains and spacer and transmembrane domains are selected so that each polypeptide chain can be expressed independently and will remain unassociated with the other in the absence of ligand.

2. DNA according to Claim 1 wherein each intracellular domain coded for is a naturally occurring polypeptide signalling sequence.

3. DNA according to Claim 2 wherein each intracellular domain is all or part of the zeta, eta or epsilon chain derived from the T-cell receptor; CD28; CD4; CD8; or the γ chain of a Fc receptor.

4. DNA according to Claim 3 wherein the intracellular domain sequence in one chain is derived from a CD4 intracellular chain containing the lck binding domain and the intracellular domain in the other chain is a zeta chain derived from the T-cell receptor.

5. DNA according to any one of Claims 1 to 4 wherein the transmembrane domain coded for is an oligo-or polypeptide derived from all or part of the alpha, beta or zeta chain of the T-cell receptor, CD28, CD8, CD4, CD3ε or CD45.

6. DNA according to any one of Claims 1 to 5 wherein each spacer domain is a polypeptide comprising 20 to 100 amino acids.

7. DNA according to Claim 6 wherein the spacer and/or transmembrane domains have been modified by deleting, changing or otherwise modifying amino acids which have side chains capable of covalently or non-covalently interacting with the side chains of amino acids in the other chain.

8. DNA according to Claim 7 wherein the amino acids that have been modified are selected from cysteine residues, charged amino acids or amino acids within potential glycosylation sites.

9. DNA according to any one of Claims 1 to 8 wherein each independent polypeptide chain coded for additionally has a secretion signal sequence attached to the N-terminus of the association domain of each chain.

10. DNA according to any one of Claims 1 to 9 in association with a carrier.

11. DNA according to Claim 10 wherein the carrier is a viral vector, a liposomal vector, a cationic lipid or an antibody.

12. DNA according to Claim 11 wherein the carrier is a targeted carrier.

13. DNA according to any one of Claims 1 to 12 which is located on a plasmid.

14. DNA according to claim 13 which is located on plasmid pHMF374 as described in Figure 3 herein.

15. An isolated effector cell containing DNA or a plasmid according to any one of Claims 1 to 14.

## Patentansprüche

1. DNA, welche für einen chimären Rezeptor kodiert, der zwei unabhängige Polypeptidketten enthält, wobei jede der Ketten in einer N- zu C-Terminus-Sequenz
(1) eine extrazelluläre Ligandenassoziations-Domäne,
(2) eine Spacer-Domäne,
(3) eine Transmembran-Domäne und
(4) eine oder mehrere intrazelluläre Domänen
umfasst,
mit der Maßgabe, dass
die Ligandenassoziations-Domäne einer Polypeptidkette des kodierten chimären Rezeptors eine V_{H}-Domäne oder ein Liganden-bindendes Fragment davon ist und die Ligandenassoziations-Domäne der anderen Polypeptidkette des kodierten chimären Rezeptors eine V_{L}-Domäne oder ein Liganden-bindendes Fragment davon ist;
mindestens zwei der Domänen in einer Kette nicht natürlich miteinander fusioniert sind;
und
die Ligandenassoziations-Domänen und Spacer- und Transmembran-Domänen ausgewählt sind, so dass jede Polypeptidkette unabhängig exprimiert werden kann und in Abwesenheit von Ligand mit der anderen unassoziiert bleibt.

2. DNA nach Anspruch 1, wobei jede kodierte intrazelluläre Domäne eine natürlich vorkommende Polypeptid-Signalsequenz ist.

3. DNA nach Anspruch 2, wobei jede intrazelluläre Domäne das Gesamte oder ein Teil der von dem T-Zell-Rezeptor abgeleiteten Zeta-, Eta- oder Epsilon-Kette, CD28, CD4, CD8, oder die γ-Kette eines Fc-Rezeptors ist.

4. DNA nach Anspruch 3, wobei die intrazelluläre Domänen-Sequenz in einer Kette von einer CD4 intrazellulären Kette abgeleitet ist, welche die Ick Bindungs-Domäne enthält, und die intrazelluläre Domäne in der anderen Kette eine von dem T-Zell-Rezeptor abgeleitete Zeta-Kette ist.

5. DNA nach einem der Ansprüche 1 bis 4, wobei die kodierte Transmembran-Domäne ein Oligo- oder Polypeptid ist, welches von dem Gesamten oder einem Teil der Alpha-, Beta- oder Zeta-Kette des T-Zell-Rezeptors, CD28, CD8, CD4, CD3ε oder CD45 abgeleitet ist.

6. DNA nach einem der Ansprüche 1 bis 5, wobei jede Spacer-Domäne ein Polypeptid ist, welches 20 bis 100 Aminosäuren umfasst.

7. DNA nach Anspruch 6, wobei die Spacer- und/oder Transmembran-Domänen durch Deletieren, Ändern oder anderweitiges Modifizieren von Aminosäuren, welche Seitenketten aufweisen, die in der Lage sind, kovalent oder nichtkovalent mit den Seitenketten von Aminosäuren in der anderen Kette wechselzuwirken, modifiziert worden sind.

8. DNA nach Anspruch 7, wobei die Aminosäuren, welche modifiziert wurden, aus Cystein-Resten, geladenen Aminosäuren oder Aminosäuren mit potenziellen Glycosylierungsstellen ausgewählt sind.

9. DNA nach einem der Ansprüche 1 bis 8, wobei jede kodierte unabhängige Polypeptidkette zusätzlich eine an den N-Terminus der Assoziations-Domäne jeder Kette gebundene Sekretionssignal-Sequenz aufweist.

10. DNA nach einem der Ansprüche 1 bis 9 in Assoziation mit einem Träger.

11. DNA nach Anspruch 10, wobei der Träger ein viraler Vektor, ein liposomaler Vektor, ein kationisches Lipid oder ein Antikörper ist.

12. DNA nach Anspruch 11, wobei der Träger ein gerichteter Träger ist.

13. DNA nach einem der Ansprüche 1 bis 12, welche auf einem Plasmid angeordnet ist.

14. DNA nach Anspruch 13, welche auf Plasmid pHMF374, wie hierin in Figur 3 beschrieben, angeordnet ist.

15. Isolierte Effektor-Zelle, welche DNA oder ein Plasmid nach einem der Ansprüche 1 bis 14 enthält.

## Revendications

1. ADN codant pour un récepteur chimère contenant deux chaînes polypeptidiques indépendantes, chacune desdites chaînes comprenant, dans une séquence N- à C-terminale :
(1) un domaine d'association de ligand extracellulaire ;
(2) un domaine espaceur ;
(3) un domaine transmembranaire ; et
(4) un ou plusieurs domaines intracellulaires ;
du moment que :
le domaine d'association de ligand d'une chaîne polypeptidique du récepteur chimère codé est un domaine V_{H}, ou un fragment de liaison de ligand de ce dernier, et le domaine d'association de ligand de l'autre chaîne polypeptidique du récepteur chimère codé est un domaine V_{L} ou un fragment de liaison de ligand de ce dernier ;
au moins deux desdits domaines d'une chaîne ne sont pas naturellement fusionnés l'un à l'autre ; et
les domaines d'association de ligand et les domaines espaceur et transmembranaire sont choisis de façon que chaque chaîne polypeptidique puisse être exprimée d'une manière indépendante, et reste non associée à l'autre en l'absence de ligand.

2. ADN selon la revendication 1, dans lequel chaque domaine intracellulaire codé est une séquence signal polypeptidique naturelle.

3. ADN selon la revendication 2, dans lequel chaque domaine intracellulaire représente tout ou partie de la chaîne zêta, êta ou epsilon dérivée du récepteur des cellules T ; du CD28 ; du CD4 ; du CD8 ; ou la chaîne γ d'un récepteur Fc.

4. ADN selon la revendication 3, dans lequel la séquence du domaine intracellulaire d'une chaîne dérive d'une chaîne intracellulaire du CD4 contenant le domaine de liaison lck, et le domaine intracellulaire de l'autre chaîne est une chaîne zêta dérivée du récepteur des cellules T.

5. ADN selon l'une quelconque des revendications 1 à 4, dans lequel le domaine transmembranaire codé est un oligo- ou polypeptide qui dérive de tout ou partie de la chaîne alpha, bêta ou zêta du récepteur des cellules T, du CD28, du CD8, du CD4, du CD3ε ou du CD45.

6. ADN selon l'une quelconque des revendications 1 à 5, dans lequel chaque domaine espaceur est un polypeptide comprenant 20 à 100 acides aminés.

7. ADN selon la revendication 6, dans lequel le domaine espaceur et/ou le domaine transmembranaire ont été modifiés par délétion, changement ou une autre modification d'acides aminés qui possèdent des chaînes latérales capables d'interagir d'une manière covalente ou non covalente avec les chaînes latérales des acides aminés de l'autre chaîne.

8. ADN selon la revendication 7, dans lequel les acides aminés qui ont été modifiés sont choisis parmi les résidus de cystéine, les acides aminés chargés ou les acides aminés se trouvant dans les sites potentiels de glycosylation.

9. ADN selon l'une quelconque des revendications 1 à 8, dans lequel chaque chaîne polypeptidique indépendante codée possède en outre une séquence signal de sécrétion fixée au site N-terminal du domaine d'association de chaque chaîne.

10. ADN selon l'une quelconque des revendications 1 à 9 en association avec un support.

11. ADN selon la revendication 10, dans lequel le support est un vecteur viral, un vecteur liposomal, un lipide cationique ou un anticorps.

12. ADN selon la revendication 11, dans lequel le support est un support ciblé.

13. ADN selon l'une quelconque des revendications 1 à 12, qui est situé sur un plasmide.

14. ADN selon la revendication 13, qui est situé sur le plasmide pHMF374 tel que décrit dans la Figure 3 de la présente invention.

15. Cellule effectrice isolée contenant un ADN ou un plasmide selon l'une quelconque des revendications 1 à 14.


### SEQUENCE LISTING

<110> CELLTECH THERAPEUTICS LIMITED
<120> CHIMERIC RECEPTORS
<130> P021625WO/CPM
<140> PCT/GB99/01417
   <141> 1999-05-06
<160> 14
<170> PatentIn Ver. 2.1
<210> 1
   <211> 30
   <212> DNA
   <213> Artificial Sequence
<220>
   <223> Description of Artificial Sequence: oligo S4501 from Figure 2
<400> 1
   cgcaagcttg ccgccaccat ggaatggagc 30
<210> 2
   <211> 30
   <212> DNA
   <213> Artificial Sequence
<220>
   <223> Description of Artificial Sequence: oligo S4502 from Figure 2
<400> 2
   tggactagtt gaggcagaag acactgtcac 30
<210> 3
   <211> 29
   <212> DNA
   <213> Artificial Sequence
<220>
   <223> Description of Artificial Sequence: oligo S4503 from Figure 2
<400> 3
   cgcaagcttg ccgccaccat gtctgtccc 29
<210> 4
   <211> 32
   <212> DNA
   <213> Artificial Sequence
<220>
   <223> Description of Artificial Sequence: oligo S4504 from Figure 2
<400> 4
   tggactagtc gtacgtttta cttctacttt ag 32
<210> 5
   <211> 70
   <212> DNA
   <213> Artificial Sequence
<220>
   <223> Description of Artificial Sequence: oligo S4881 from Figure 2
<400> 5
   cgtgccggtc ttcctgccag cgaagcccgg tgcggggcca gcgccgcgac caccaacacc 60
   ggcgcccacc                                                        70
<210> 6
   <211> 72
   <212> DNA
   <213> Artificial Sequence
<220>
   <223> Description of Artificial Sequence: oligo S4882 from Figure 2
<400> 6
   gaggcggcgc ggccagcggc ggggggcgca gtgcacacga gggggctgga cttcgcggcc 60
   gccctgattg tg 72
<210> 7
   <211> 72
   <212> DNA
   <213> Artificial Sequence
<220>
   <223> Description of Artificial Sequence: oligo S4883 from Figure 2
<400> 7
   cgccgctggc cgcgccgcct ctgggcgcag ggacaggggc tgcgacgcga tggtgggcgc 60
   cggtgttggt gg 72
<210> 8
   <211> 65
   <212> DNA
   <213> Artificial Sequence
<220>
   <223> Description of Artificial Sequence: oligo S4884 from Figure 2
<400> 8
   cgctggcagg aagaccggca cgaagtggct gaagtacatg atggagttgc tcagggcact 60
   agttg 65
<210> 9
   <211> 23
   <212> DNA
   <213> Artificial Sequence
<220>
   <223> Description of Artificial Sequence: oligo S4885 from Figure 2
<400> 9
   caactagtgc cctgagcaac tcc 23
<210> 10
   <211> 22
   <212> DNA
   <213> Artificial Sequence
<220>
   <223> Description of Artificial Sequence: oligo S4886 from Figure 2
<400> 10
   cacaatcagg gcggccgcga ag 22
<210> 11
   <211> 39
   <212> DNA
   <213> Artificial Sequence
<220>
   <223> Description of Artificial Sequence: oligo S4499 from Figure 2
<400> 11
   ctgcagttcg cggccgccct gattgtgctg gggggcgtc 39
<210> 12
   <211> 45
   <212> DNA
   <213> Artificial Sequence
<220>
   <223> Description of Artificial Sequence: oligo S4500 from Figure 2
<400> 12
   gccgagctcc tatatgaatt ctcaaatggg gctacatgtc ttctg 45
<210> 13
   <211> 34
   <212> DNA
   <213> Artificial Sequence
<220>
   <223> Description of Artificial Sequence: oligo S4700 from Figure 2
<400> 13
   tatgaattct tagcgagggg gcagggcctg catg 34
<210> 14
   <211> 101
   <212> DNA
   <213> Artificial Sequence
<220>
   <223> Description of Artificial Sequence: oligo S4701 from Figure 2
<400> 14
   ctggacttcg cggccgccct gattgtgctg gggggcgtcg ccggcctcct gcttttcatt 60
   gggctaggca tcttcttcag agtgaagttc agcaggagcg c                     101